# EUROPÄISCHE PATENTSCHRIFT

(11) **EP 2 015 786 B1**
(45) Veröffentlichungstag und Bekanntmachung des Hinweises auf die Patenterteilung: **30.03.2011**
(21) Anmeldenummer: 07724018.2
(22) Anmeldetag: 05.04.2007
(51) Int. Cl.: A61L 15/26, A61L 15/42

(54) **BIOMEDIZINISCHE SCHAUMARTIKEL**
BIOMEDICAL FOAM ARTICLES
ARTICLES MOUSSANTS BIOMÉDICAUX

(30) Priorität: 08.04.2006 DE 102006016636
(43) Veröffentlichungstag der Anmeldung: 21.01.2009
(73) Patentinhaber: Bayer MaterialScience AG, 51368 Leverkusen (DE)
(72) Erfinder: DIETZE, Melita, 40699 Erkrath (DE); FUGMANN, Burkhard, 40878 Ratingen (DE); MAGER, Michael, 51375 Leverkusen (DE); RISCHE, Thorsten, 59423 Unna (DE); HECKES, Michael, 47800 Krefeld (DE); RUDHARDT, Daniel, 50823 Köln (DE); GERTZMANN, Rolf, 51377 Leverkusen (DE)
(86) Internationale Anmeldenummer: PCT/EP2007/003077
(87) Internationale Veröffentlichungsnummer: WO 2007/115781

(56) Entgegenhaltungen:
- WO-A-2004/037307
- WO-A-2007/115696
- US-B1- 6 605 666

## Beschreibung

Die vorliegende Erfindung betrifft biomedizinische Schaumartikel für den Wundbereich, die durch Aufsprühen eines Polymers auf eine Wunde gewonnen werden. Das Polymer ergibt während des Aufsprühens auf eine Wundoberfläche einen sich der räumlichen Form der Wunde anpassenden dreidimensionalen Formkörper, der neben der Abdeckung der Wundoberfläche, eine vollständige und paßgenaue Auskleidung der Wunde auch in der Tiefendimension gewährleistet und darüber hinaus stark absorbierende Eigenschaften hat. Die erfindungsgemäßen biomedizinischen Schaumartikel eignen sich insbesondere zur Behandlung chronischer Wunden.

Als chronische Wunden werden alle Wunden bezeichnet, die nicht innerhalb einer physiologischen Abheilzeit von 2-3 Wochen epithelisiert sind. Die mit Abstand häufigsten Formen chronischer Wunden sind Dekubitalulzera (verursacht durch chronische Druckbelastung), chronisch-venöse Ulzera der Beine (verursacht durch chronisch venöse Insuffizienz) und diabetische Ulzera (verursacht durch Angiopathie und Neuropathie).

Die Standardbehandlung chronischer Wunden folgt dem Prinzip der "feuchten Wundheilung" mit unterschiedlichen Wundauflagenmaterialien. Die typischen Materialien der feuchten Wundbehandlung werden als Vliese auf die Wunde aufgelegt, um eine optimale Wundabdeckung zu erhalten und durch Aufrechterhaltung des feuchten Wundmilieus die Wundheilung zu beschleunigen.

Die Erstreckung konventioneller Behandlungsmethoden auf chronische Wunden weist jedoch den Nachteil auf, dass konventionelle Wundauflagen lediglich die Wundoberfläche abdecken, die Wunde jedoch nicht dreidimensional (vor allem in der Tiefe) auskleiden, was zu Defiziten beim Exudathandling, erhöhtem Infektionsrisiko aber auch verstärkter Mazeration an den Wundränder führen kann.

Eine fehlende Wundauskleidung kann z.B. bei cavitären Wunden zu Exudatansammlungen am Wundgrund führen, was neben einer Behinderung der Wundheilung auch zu einem Aufweichen des gesunden Gewebes am Wundrand und letztlich zur Mazeration führt. Das Vorhandensein von überschüssigem Exudat begünstigt weiterhin das Entstehen von Infektionen.

EP 171 268 B1 offenbart einen Wundverband, welcher eine Vielzahl von Stücken eines saugfähigen Materials in einer porösen Tasche aufweist. Nachteilig an einem solchen ist allerdings, dass er nicht immer zu einer paßgenauen Auskleidung der Wunde auch in der Tiefendimension führt. Weiterhin ist ein solcher Wundverband umständlich zu handhaben und unter Umständen nur schwierig steril zu halten.

DE 36 38 379 offenbart ein Verfahren zur Herstellung eines medizinischen Wundverbandes basierend auf einer bei Raumtemperatur härtenden, zweikomponentigen Polyorganosiloxanzusammensetzung, wodurch eine elastische Polysiloxanschaummasse erhalten wird, die sich den Konturen einer Wunde anpassen kann. Die so gebildete Polysiloxanschaummasse ist jedoch nicht stark absorbierend und kann daher für Wunden, welche große Mengen Wundflüssigkeit absondern, nicht eingesetzt werden.

Es besteht daher ein Bedarf an einer neuartigen Wundauflage, die sich den oftmals tiefen und/oder komplexen Wundformen, die für viele chronische Wunden typisch sind, durch ihre in Fläche und Tiefe anpassende Formung optimal anpasst. Eine solche Wundauflage sollte weiterhin einfach und hygienisch aufzutragen sein sowie vorzugsweise antibakterielle, schmerzlindernde und/oder wundheilungsbeschleunigenda Wirkung entfalten. Weitere wichtige Eigenschaften sind eine schnelle Aushärtung sowie eine ausreichende Flüssigkeitsausnahme (Absorption) des die Wundauflage bildenden Materials.

Voraussetzung für einen effektiven Einsatz ist eine schnelle Aushärtung (d.h. Verfestigung des flüssigen Polymers zu einem festen Schaumartikel, festgestellt durch sensorische Überprüfung der Viskosität) des biomedizinischen Schaumartikels in einer Zeit von nicht mehr als fünf Minuten, bevorzugt nicht mehr als 2 Minuten, besonders bevorzugt nicht mehr als einer Minute, ganz besonders bevorzugt weniger als 30 Sekunden.

Weitere Vorraussetzung ist ein Absorptionsvermögen gegenüber physiologischer Kochsalzlösung von 100 bis 2.500 %, bevorzugt 100 % bis 2.000 %, besonders bevorzugt 100 bis 1500 % und ganz besonders bevorzugt 300 bis 1500 % (Bestimmung nach DIN EN 13726-1, Teil 3.2) sowie eine Durchlässigkeit gegenüber Wasserdampf von 2.000 bis 12.000 g/m² in 24 h, bevorzugt 3.000 bis 10.000 g/m² in 24 h, besonders bevorzugt 3.000 bis 5000 g/m² in 24 h (Bestimmung nach DIN EN 13726-2, Teil 3.2). Hierzu ist erforderlich, dass Schaum eine zumindest teilweise Offenzelligkeit aufweist.

Es wurde nun überraschend gefunden, dass diese Aufgabe durch die nachstehend beschriebenen erfindungsgemäßen biomedizinischen Schaumartikel gelöst wird.

Ein erster Gegenstand der vorliegenden Erfindung ist ein biomedizinischer Schaumartikel erhältlich durch das Ansprühen einer Zusammensetzung umfassend mindestens eine ionische Polymerdispersion oder -emulsion, mindestens ein Koagulans und ein Schaumhilfsmittel sowie optional mindestens einen Wirkstoff ausgewählt aus der Gruppe der Breitbandantibiotika, Antiseptika, der antiviralen Wirkstoffe, der antifungischen Wirkstoffe, der antipathogenen Peptide, der Lokalanästhetika, der nichtsteroidaten Antiphlogistika, der Opiate sowie der hämostatisch, wundheilend, granulationsfördernd wirkenden Wirkstoffe auf ein Sübstrat.

Ein bevorzugtes Substrat ist die menschliche oder tierische Haut, welche ein oder mehrere Wundstellen aufweist..

Die Vorteile der Sprühapplikation liegen insbesondere in der Handhabung des Produktes. Durch die Applikation einer Fertiglösung aus einer sterilen Spraydose entfällt das Entpacken, Zurechtschneiden und Auflegen der herkömmlichen Materialien d.h. die Applikation kann sogar durch den Patienten selbst durchgeführt werden, beschleunigt den Vorgang des Verbandwechsels und ist hygienischer, da der direkte manuelle Kontakt mit der Wunde beim Verbandswechsel entfällt.

Bevorzugt sind ionische Polymerdispersionen, welche als kontinuierliche Phase ein wasserhaltiges Medium besitzen.

Geeignete ionische Polymerdispersionen der vorstehend genannten Art sind beispielsweise ionische Kautschuk-Latex-Dispersionen, ionische Polyurethan-Dispersionen, Dispersionen ionischer (Meth)acrylat-Copolymere sowie Dispersionen natürlich vorkommender ionischer Biopolymere auf Kohlenhydrat-Basis wie Cellulosederivate, z. B. Celluloseacetatphthalat (CAP), Cellutoseacetatsuccinat (CAS), Celluloseacetattrimelitat (CAT), Hydroxypropylmethylcellulosephthalat (HPMCP), Carboxymethylcellulose (CMC), Chitosan, auch Chitin, Hyaluronan, Cellulose, Dextrin oder Stärke sowie weiterer natürlicher Biopolymere wie z.B. Lignin oder Casein.

Geeignetes (Meth)acrylat-Copolymer ist bevorzugt ein (Meth)acrylat-Copolymer aus 40 bis 95 Gew.% radikalisch polymerisierten C₁- bis C₄-Alkylestern der Acryl- oder der Methacrylsaeure und enthaltend 5 bis 60 Gew.-% (Meth)acrylat-Monomeren mit einer anionischen Gruppe im Alkylrest Das (Meth)acrylat-Copolymere besteht zu 40 bis 100, bevorzugt zu 45 bis 99, insbesondere zu 85 bis 95 Gew.% aus radikalisch polymerisierten C₁- bis C₄-Alkylestern der Acryl- oder der Methacrylsäure und kann 0 bis 60, bevorzugt 1 bis 55, insbesondere 5 bis 15 Gew.-% (Meth)acrylat-Monomere mit einer anionischen Gruppe im Alkylrest enthalten.

In der Regel addieren sich die genannten Anteile zu 100 Gew.-%. Es können jedoch zusätzlich, ohne dass dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Hydroxyethylmethacrylat oder Hydroxyethylacrylat enthalten sein.

Bevorzugte ionische Polymerdispersionen sind wässrige, ionische Polyurethan-Dispersionen, aliphatische Polyurethan-Dispersionen sowie Polyurethan Hybrid Emulsionen. Besonders bevorzugte Polymerdispersionen sind wässrige, anionische, hydrophile Polyurethan -Dispersionen.

Ganz besonders bevorzugt sind wässrige, anionische, hydrophile Polyurethan -Dispersionen , erhältlich, indem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
   A3) gegebenenfalls hydroxyfunktionellen Verbindungen mit Molekulargewichten von 62 bis 399 g/mol und
   A4) gegebenenfalls isocyanatreaktiven, anionischen oder potentiell anionischen und/oder gegebenenfalls nichtionischen Hydrophilierungsmitteln,
   hergestellt werden,
B) deren freie NCO-Gruppen dann ganz oder teilweise
B1) gegebenenfalls mit aminofunktionellen Verbindungen mit Molekulargewichten von 32 bis 400 g/mol und
B2) mit isocyanatreaktiven, bevorzugt aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

Um eine anionische Hydrophilierung zu erreichen müssen in A4) und/oder B2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO⁻ oder - SO₃- oder -PO₃²⁻ als anionische bzw. deren ganz oder teilweise protonierte Säureformen als potentiell anionische Gruppen aufweisen.

Bevorzugte wässrige, anionische Polyurethan-Dispersionen (I) haben einen niedrigen Grad an hydrophilen anionischen Gruppen, bevorzugt von 0,1 bis 15 Milliequivalenten pro 100 g Festharz.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie.

Das Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) beträgt bei der Herstellung des NCO-funktionellen Prepolymers 1,05 bis 3,5, bevorzugt 1,2 bis 3,0 besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt zwischen 50 bis 125 %, besonders bevorzugt zwischen 60 bis 120 % beträgt.

Geeignete Polyisocyanate der Komponente A1) sind die dem Fachmann an sich bekannten aromatischen, araliphatischen, aliphatischen oder cycloaliphatischen Polyisocyanate einer NCO-Funktionalität von ≥ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenyleniisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis-(isocyanatomethyl)benzol (XDI), sowie Alkyl-2,6-diisocyanatohexanoate (Lysindiisocyanate) mit C₁-C₈-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül sei z.B. 4-Isocyanatomethyl-1,8-octandiisocyanat (Nonantriisocyanat) oder Triphenylmethan-4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in A1) 1,6-Hexamethylendiisocyanat, Isophorondiisocyanat, die isomeren Bis(4,4'-isocyanatocyclohexyl)methane, sowie deren Mischungen eingesetzt.

In A2) werden polymere Polyole mit einem zahlenmittleren Molekulargewicht Mₙ von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Triemthylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol, bevorzugt 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Die Hydroxylgruppen aufweisenden Polycarbonate sind bevorzugt linear gebaut.

Ebenfalls können in A2) Polyetherpolyole eingesetzt werden.

Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrin an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan Dispersionen (I) enthalten als Komponente A2) eine Mischung aus Polycarbonatpolyolen und Polytetramethylenglykolpolyolen, wobei in dieser Mischung der Anteil an Polycarbonatpolyolen in der Mischung 20 bis 80 Gew.-% und der Anteil an Polytetramethylenglykolpolyolen 80 bis 20 Gew.-% beträgt. Bevorzugt ist ein Anteil von 30 bis 75 Gew.-% an Polytetramethylenglykolpolyolen und ein Anteil von 25 bis 70 Gew.-% an Polycarbonatpolyolen. Besonders bevorzugt ist ein Anteil von 35 bis 70 Gew.-% an Polytetramethylenglykolpolyolen und ein Anteil von 30 bis 65 Gew.-% an Polycarbonatpolyolen, jeweils mit der Maßgabe, dass die Summe der Gewichtsprozente der Polycarbonat- und Polytetramethylenglykolpolyole 100 % ergibt und der Anteil der Summe der Polycarbonat- und Polytetramethylenglykolpolyetherpolyole an der Komponente A2) mindestens 50 Gew.-%, bevorzugt 60 Gew.-% und besonders bevorzugt mindestens 70 Gew.-% beträgt.

Die Verbindungen der Komponente A3) besitzen Molekulargewichte von 62 und 400 g/mol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-εhydroxycapronsäureester, ω-Hydroxyhexyl-γ-hydroxybuttersäure-ester, Adipinsäure-(β-hydroxyethyl)ester oder Terephthalsäurebis(β-hydroxyethyl)-ester.

Ferner können in A3) auch monofunktionelle, isocyanatreaktive, Hydroxylgruppen-haltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykol-monomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugte Verbindungen der Komponente A3) sind 1,6-Hexandiol. 1,4-Butandiol, Neopentylglykol und Trimethylolpropan.

Unter anionisch bzw. potentiell anionisch hydrophilierenden Verbindungen der Komponente A4) werden sämtliche Verbindungen verstanden, die mindestens eine isocyanatreaktive Gruppe wie eine Hydroxylgruppe aufweisen sowie mindestens eine Funktionalität wie z.B. -COO⁻M⁺, -SO₃⁻ M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5-9, Formel I-III beschrieben ist. Bevorzugte anionische oder potentiell anionische Hydrophilierungsmittel der Komponente A4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel sind solche, die Carboxylat- bzw Carbonsäuregruppen als ionische oder potentiell ionische Gruppen enthalten, wie Dimethylolpropionsäure, Dimethylolbuttersäue und Hydroxypivalinsäure bzw. deren Salze.

Geeignete nichtionisch hydrophilierende Verbindungen der Komponente A4) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Starterinoleküle zugänglich sind (z.B. in Ullmanns Encyclopädie der technischen Chemie, 4. Auflage, Band 19, Verlag Chemie, Weinheim S. 31-38).

Diese sind entweder reine Polyethylenoxidether oder gemischte Polyalkylenoxidether, wobei sie mindestens 30 mol-%, bevorzugt mindestens 40 mol% bezogen auf alle enthaltenen Alkylenoxideinheiten an Ethylenoxideinheiten enthalten.

Bevorzugte Polyethylenoxidether der vorstehend genannten Art sind monofunktionelle gemischte Polyalkylenoxidpolyether, die 40 bis 100 mol% Ethylenoxid- und 0 bis 60 mol% Propylenoxideinheiten aufweisen.

Bevorzugte nichtionisch hydrophilierende Verbindungen der Komponente A4) sind solche der vorstehend genannten Art, wobei es sich um Block(co)polymere handelt, die durch blockweise Addition von Alkylenoxiden an geeignete Starter hergestellt werden.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Düsopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin oder sowie Hydraziden wie Adipinsäuredihydrazid.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- Methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente B1) sind 1,2-Ethylendiamin, 1,4-Diaminobutan und Isophorondiamin.

Unter anionisch bzw. potentiell anionisch hydrophilierenden Verbindungen der Komponente B2) werden sämtliche Verbindungen verstanden, die mindestens eine isocyanatreaktive Gruppe, bevorzugt eine Amino-Gruppe aufweisen, sowie mindestens eine Funktionalität wie z.B. -COO⁻ M⁺, -SO₃⁻M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, der bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Amino-ethylamino)-ethansulfonsäure, Ethylendiamin-propyl- oder -butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1); Weiterhin kann Cyclohexylaminopropansulfonsäure (CAPS) aus WO-A 01/88006 als anionisches oder potentiell anionisches Hydrophilierungsmittel verwendet werden.

Bevorzugte anionische oder potentiell anionische Hydrophilierungsmittel der Komponente B2) sind solche der vorstehend genannten Art, die über Carboxylat- bzw. Carobonsäuregruppen und/oder Sulfonatgruppen verfügen wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und B1)
0,1 bis 25 Gew.-% Summe der Komponenten Komponente A4) und B2), wobei bezogen auf die Gesamtmengen der Komponenten A1) bis A4) und B1) bis B2) 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und B1)
0,1 bis 15 Gew.-% Summe der Komponenten Komponente A4) und B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1
65 bis 85 Gew.-% A2),
0,5 bis 14 Gew.-% Summe der Komponenten A3) und B1)
0,1 bis 13,5 Gew.-% Summe der Komponenten A4) und B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der anionisch hydrophilierten Polyurethan-Dispersionen (I) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z.B. Prepolymer-Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu mit Isocyanat reaktiven Gruppen 1,05 bis 3,5, bevorzugt 1,2 bis 3,0, besonders bevorzugt 1,3 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält. Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerten Polyurethanpolymerlösungen gerührt. Bevorzugt wird das Wasser in das gelöste kettenverlängerte Polyurethan polymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (I) beträgt typischerweise weniger als 1,0 Gew.-%, bevorzugt weniger als 0,5 Gew.-%, bezogen auf die gesamte Dispersion.

Der pH-Wert der erfindungswesentlichen Polyurethan-Dispersionen (I) beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Dispersionen (I) beträgt 40 bis 70, bevorzugt 50 bis 65, besonders bevorzugt 55 bis 65 Gew.-%.

**Die für die tatsächlich verwendete Polymerdispersion oder -emulsion jeweils geeigneten Koagulantien sind die aus der Literatur bekannten; diese sind dem Fachmann geläufig.**

Als Koagulantien (II) werden typischerweise mindestens 2 kationische Gruppen enthaltende organische Verbindungen, bevorzugt alle bekannten kationischen Flockulations- und Fällungsmittel des Stands der Technik, wie kationische Homo- oder Copolymere von Salzen des Poly[2-(N,N,N-trimethylamino)-ethylacrylats], Polyethylenimins, Poly[N-(dimethylaminomethyl)acrylamids], substituierter Acrylamide, substituierter Methacrylamide, N-Vinylformamids, N-Vinylacetamids, N-Vinylimidazols, 2-Vinylpyridins oder 4-Vinylpyridins eingesetzt.

Bevorzugte kationische Koagulanzien (II) sind Copolymere des Acrylamids, welche Struktureinheiten der allgemeinen Formel (2), besonders bevorzugt der allgemeinen Formel (1) und (2) aufweisen 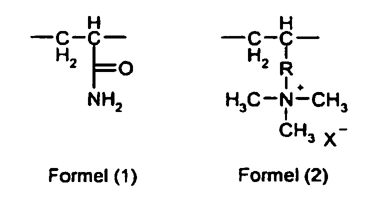 wobei
- R: C=O, -COO(CH₂)₂- oder -COO(CH₂)₃- und
- X⁻: ein Halogenidion, bevorzugt Chlorid ist

Bevorzugt weisen die Koagulazien (II) zahlenmittlere Molekulargewichte von 500.000 bis 50.000.000 g/mol auf.

Solche Koagulantien (II) werden beispielsweise unter dem Markennamen Praestol^{®} (Degussa Stockhausen, Krefeld, DE) als Flockungsmittel für Klärschlämme vertrieben. Bevorzugte Koagulantien vom Praestol^{®}-Typ sind Praestol^{®} K111L, K122L, K133L, BC 270L, K 144L, K 166L, BC 55L, 185K, 187K, 190K, K222L, K232L, K233L, K234L, K255L, K332L, K 333L, K 334L, E 125, E 150 sowie deren Mischungen. Ganz besonders bevorzugte Koagulationsmittel sind Praestol^{®} 185K, 187K und 190K sowie deren Mischungen.

Die Restgehalte an Monomeren, insbesondere Acrylat- und Acrylamidmonomere, liegen bei den Koagulantien vorzugsweise bei weniger als 1 Gew.-%, besonders bevorzugt bei weniger als 0,5 Gew.-% und ganz besonders bevorzugt bei weniger als 0,025 Gew.-%.

Die Koagulantien können in fester Form oder als wässrige Lösungen bzw. Dispersionen eingesetzt werden. Bevorzugt ist die Verwendung von wässrigen Dispersionen bzw. Lösungen.

Schaumhilfsmittel wie Schaumbildner und - stabilisatoren werden bevorzugt eingesetzt. Geeignet sind beispielsweise handelsübliche Verbindungen wie Fettsäureamide, Sulfosuccinamide, Kohlenwasserstoffsulfonate, -sulfate oder Fettsäuresalze, wobei der lipophile Rest bevorzugt 12 bis 24 Kohlenstoffatome enthält.

Bevorzugte Schaumhilfsmittel sind Alkansulfonate oder-sulfate mit 12 bis 22 Kohlenstoffatomen im Kohlenwasserstoffrest, Alkylbenzosulfonate oder -sulfate mit 14 bis 24 Kohlenstoffatomen im Kohlenwasserstoffrest oder Fettsäureamide oder Fettsäuresalze mit 12 bis 24 Kohlenstoffatomen.

Solche Fettsäureamide sind vorzugsweise solche auf Basis von Mono- oder Di-(C₂₋₃-alkanol)aminen. Die Fettsäuresalze können beispielsweise Alkalimetallsalze, Aminsatze oder unsubstituierte Ammoniumsalze sein.

Solche Fettsäurederivate basieren typischerweise auf Fettsäuren wie Laurinsäure, Myristinsäure, Palmitinsäure, Ölsäure, Stearinsäure, Ricinolsäure, Behensäure oder Arachidinsäure, Kokosfettsäure, Talgfettsäure, Sojafettsäure und deren Hydrierungsprodukten.

Besonders bevorzugte Schaumhüfsmittel sind Mischungen aus Sulfosuccinamiden und Ammoniumstearaten, wobei diese bevorzugt 20 bis 60 Gew.-% besonders bevorzugt 30 bis 50 Gew.-% an Ammoniumstearaten und bevorzugt 80 bis 40 Gew.-%, besonders bevorzugt 70 bis 50 Gew.-% an Sulfosuccinamiden enthalten.

Neben den Polyurethan-Dispersionen (I) und den Koagulantien (II) können auch weitere Hilfs- und Zusatzstoffe (III) mitverwendet werden.

Beispiele für solche Hilfs- und Zusatzstoffe (III) sind,

Verdicker bzw. Thixotropiermittel, Antioxidantien, Lichtschutzmittel, Emulgatoren, Weichmacher, Pigmente, Füllstoffe und/oder Verlaufshilfsmittel.

Als Verdicker können handelsübliche Verdicker eingesetzt werden, wie Dextrin-, Stärke- oder Cellulosederivate wie Celluloseether oder Hydroxyethylcellulose, organische vollsynthetische Verdicker, auf Basis von Polyacrylsäuren, Polyvinylpyrrolidonen, Poly(meth)acrylverbindungen oder Polyurethanen (assoziative Verdicker) sowie anorganische Verdicker, wie Bentonite oder Kieselsäuren.

Grundsätzlich können, wenn auch nicht bevorzugt, die erfindungswesentlichen Zusammensetzungen auch Vernetzer wie unblockierte Polyisocyanate, Amid- und Amin-Formaldehydharze, Phenolharze, Aldehyd- und Ketonharze, wie z.B. Phenol-Formaldehydharze, Resole, Furanharze, Harnstoffharze, Carbamidsäureesterharze, Triazinharze, Melaminharze, Benzoguanaminharze, Cyanamidharze oder Anilinharze enthalten.

Die erfindungswesentlichen Zusammensetzungen enthalten bezogen auf Trockensubstanz typischerweise 80 bis 99,5 Gewichtsteile der Dispersion (I), 0,5 bis 5 Gewichtsteile des kationischen Koagulans (II), 0 bis 10 Gewichtsteile Schaumhilfsmittel, 0 bis 10 Gewichtsteile Vernetzer und 0 bis 10 Gew.-% Verdicker.

Bevorzugt enthalten die erfindungswesentlichen Zusammensetzungen bezogen auf Trockensubstanz 8.5 bis 97 Gewichtsteile der Dispersion (I), 0,75 bis 4 Gewichtsteile des kationischen Koagulans (II), 0,5 bis 6 Gewichtsteile Schaumhilfsmittel, 0 bis 5 Gewichtsteile Vernetzer und 0 bis 5 Gew.-% Verdicker.

Besonders bevorzugt enthalten die erfindungswesentlichen Zusammensetzungen bezogen auf Trockensubstanz 89 bis 97 Gewichtsteile der Dispersion (I), 0,75 bis 3 Gewichtsteile des kationischen Koagulans (II), 0,5 bis 5 Gewichtsteile Schaumhilfsmittel, 0 bis 4 Gewichtsteile Vernetzer und 0 bis 4 Gewichtsteile Verdicker.

Neben den Komponenten (I), (II) und gegebenenfalls (III) können in den erfindungswesentlichen Zusammensetzungen auch andere wässrige Bindemittel eingesetzt werden. Solche wässrigen Bindemittel können z.B. aus Polyester-, Polyacrylat-, Polyepoxid- oder anderen Polyurethanpolymeren aufgebaut sein. Auch die Kombination mit strahlenhärtbaren Bindemitteln, wie sie z.B. in der EP-A-0 753 531 beschrieben sind, ist möglich. Ferner können auch andere anionische oder nicht-ionische Dispersionen, wie Polyvinylacetat-, Polyethylen-, Polystyrol-, Polybutadien-, Polyvinylchlorid-, Polyacrylat- und Copolymerisat-Dispersionen eingesetzt werden.

Das Aufschäumen im erfindungsgemäßen Verfahren geschieht durch mechanisches Rühren der Zusammensetzung bei hohen Drehzahlen oder durch Entspannung eines Treibgases.

Das mechanische Aufschäumen kann mit beliebigen mechanischen Rühr-, Misch- und Dispergiertechniken erfolgen. In der Regel wird hierbei Luft eingetragen, aber auch Stickstoff und andere Gase können hierfür benutzt werden.

Der so erhaltene Schaum wird beim Aufschäumen oder unmittelbar danach auf ein Substrat aufgetragen oder in eine Form gegeben und getrocknet.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich.

Eine befriedigende Trocknungsgeschwindigkeit der Schäume wird bereits bei 20°C beobachtet, so dass eine Trocknung auf verletztem menschlichen oder tierischen Gewebe problemlos möglich ist. Für eine schnellere Trocknung und Fixierung der Schäume werden jedoch bevorzugt Temperaturen oberhalb von 30°C benutzt. Bei der Trocknung sollten jedoch Temperaturen von 200°C bevorzugt 150°C, besonders bevorzugt 130°C nicht überschritten werden, da es anderenfalls u.a. zu unerwünschter Vergilbung der Schäume kommen kann. Möglich ist auch eine zwei- oder mehrstufige Trocknung.

Die erfindungsgemäß eingesetzte Polymerdispersion oder -emulsion kann zusätzlich physiologische aktive Stoffe in effektiven Mengen enthalten oder zugesetzt bekommen. Die erfindungsgemäßen biomedizinischen Schaumartikel können beispielsweise Lokalanästhetika, Enzyme, antibakterielle oder fungizide Wirkstoffe oder hormonelle Verbindungen enthalten.

Vorzugsweise enthält die erfindungsgemäß eingesetzte Polymerdispersion oder -emulsion mindestens einen Wirkstoff ausgewählt aus der Gruppe der Antiseptika, Wachstumsfaktoren, Proteaseinhibitoren und nichtsteroidale Antiphlogistika/Opiate.

Die erfindungsgemäßen biomedizinischen Schaumartikel eignen sich insbesondere für die Behandlung von Hautwunden, insbesondere chronische Wunden wie diabetische, venöse, Dekubitalulzera, aber auch von Verbrennungswunden und akuten Wunden, insbesondere minimal akuten Wunden.

Sie gewährleisten eine vollständige und paßgenaue Auskleidung der Wunde auch in der Tiefendimension, weisen eine schnelle Aushärtung und gute Flüssigkeitsaufnahme auf und sind einfach zu handhaben.

### Beispiele:

Sofern nicht abweichend gekennzeichnet, beziehen sich alle Prozentangaben auf das Gewicht.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

### Verwendete Substanzen und Abkürzungen:

| | |
|---|---|
| Diaminosulfonat: | NH₂-CH₂CH₂-NH-CH₂CH₂-SO₃Na (45 %ig in Wasser) |
| | |
| Desmophen^{®} C2200: | Polycarbonatpolyol, OH-Zahl 56 mg KOH/g, zahlenmittleres Molekulargewicht 2000 g/mol (BayerMaterialScience AG, Leverkusen, DE) |
| | |
| PolyTHF^{®} 2000: | Polytetramethylenglykolpolyol, OH-Zahl 56 mg KOH/g, zahlenmittleres Molekulargewicht 2000 g/mol (BASF AG, Ludwigshafen, DE) |
| | |
| PolyTHF^{®} 1000: | Polytetramethylenglykolpolyol, OH-Zahl 112 mg KOH/g, zahlenmittlereszahlenmittleres Molekulargewicht 1000 g/mol (BASF AG, Ludwigshafen, DE) |
| | |
| Polyether LB 25: | monofunktioneller Polyether auf Ethylenoxid-/Propylenoxidbasis, zahlenmittleres Molekulargewicht Leverkusen, DE) 2250 g/mol, OH-Zahl 25 mg KOH/g (BayerMaterialScience AG, |
| | |
| Stokal^{®} STA: | Schaumhilfsmittel auf Ammoniumstearat-Basis, Wirkstoffgehalt: 30 % (Bozzetto GmbH, Krefeld, DE) |
| | |
| Stokal^{®} SR: | Schaumhilfsmittel auf Succinamat-Basis, Wirkstoffgehalt: ca. 34 % (Bozzetto GmbH, Krefeld, DE) |
| | |
| Simulsol^{®} SL 26: | Alkylpolyglycosid auf Basis von Dodecylalkohol, ca. 52 %ig in Wasser, DE Seppic GmbH, Köln, |
| | |
| Praestol^{®} 185 K: | Kationisches Flockungshilfsmittel enthaltend die Strukturen der Formeln (1) und (2), Festkörpergehalt 25 % (Degussa AG, DE) |

| | |
|---|---|
| | |

Die Bestimmung der mittleren Teilchengrößen (angegeben ist das Zahlenmittel) der Polyurethan-Dispersionen (1) erfolgte mittels Laserkorrelations-Spektroskopie (Gerät: Malvern Zetasizer 1000, Malver Inst. Limited).

### Beispiel 1: Polyurethan-Dispersion 1

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht oder leicht unterschritten war. Das fertige Prepolymer wurde mit 4830 g Aceton gelöst und dabei auf 50°C abgekühlt und anschließend wurde eine Lösung aus 25,1 g Ethylendiamin, 116,5 g Isophorondiamin, 61,7 g Diaminosulfonat und 1030 g Wasser innerhalb von 10 min zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 61% |
| Partikelgröße (LKS): | 312 nm |
| Viskosität (Viskosimeter, 23°C): | 241 mPas |
| pH (23°C): | 6,02 |

### Beispiel 2: Polyurethan-Dispersion 2

223,7 g PolyTHF^{®} 2000, 85,1 g PolyTHF^{®} 1000, 172,6 g Desmophen^{®} C2200 und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht oder leicht unterschritten war. Das fertige Prepolymer wurde mit 1005 g Aceton gelöst und dabei auf 50°C abgekühlt und anschließend wurde eine Lösung aus 5,70 Ethylendiamin, 26,4 g Isophorondiamin, 9,18 g Diaminosulfonat und 249,2 g Wasser innerhalb von 10 min zudosiert. Die Nachrührzeit betrug 10 min. Dann wurde durch Zugabe von 216 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 63% |
| Partikelgröße (LKS): | 495 nm |
| Viskosität (Viskosimeter, 23°C): | 133 mPas |
| pH (23°C): | 6,92 |

### Beispiel 3: Polyurethan-Dispersion 3

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht oder leicht unterschritten war. Das fertige Prepolymer wurde mit 4830 g Aceton gelöst und dabei auf 50°C abgekühlt und anschließend wurde eine Lösung aus 36,9 g 1,4-Diaminobutan, 116,5 g Isophorondiamin, 61,7 g Diaminosulfonat und 1076 g Wasser innerhalb von 10 min zudosiert. Die Nachrührzeit betrug 10 min. Dann wurde durch Zugabe von 1210 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 59% |
| Partikelgröße (LKS): | 350 nm |
| Viskosität (Viskosimeter, 23°C): | 126 mPas |
| pH (23°C): | 7,07 |

### Beispiel 4: Polyurethan-Dispersion 4

201,3 g PolyTHF^{®} 2000, 76,6 g PolyTHF^{®} 1000, 155,3 g Desmophen^{®} C2200, 2,50 g 1,4-Butandiol und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht oder leicht unterschritten war. Das fertige Prepolymer wurde mit 1010 g Aceton gelöst und dabei auf 50°C abgekühlt und anschließend wurde eine Lösung aus 5,70 Ethylendiamin, 26,4 g Isophorondiamin, 14,0 g Diaminosulfonat und 250 g Wasser innerhalb von 10 min zudosiert. Die Nachrührzeit betrug 10 min. Dann wurde durch Zugabe von 243 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 62% |
| Partikelgröße (LKS): | 566 nm |
| Viskosität (Viskosimeter, 23°C): | 57 mPas |
| pH (23°C): | 6,64 |

### Beispiel 5: Polyurethan-Dispersion 5

201,3 g PolyTHF^{®} 2000, 76,6 g PolyTHF^{®} 1000, 155,3 g Desmophen^{®} C2200, 2,50 g Trimethylolpropan und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht oder leicht unterschritten war. Das fertige Prepolymer wurde mit 1010 g Aceton gelöst und dabei auf 50°C abgekühlt und anschließend wurde eine Lösung aus 5,70 Ethylendiamin, 26,4 g Isophorondiamin, 14,0 g Diaminosulfonat und 250 g Wasser innerhalb von 10 min zudosiert. Die Nachrührzeit betrug 10 min. Dann wurde durch Zugabe von 293 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 56% |
| Partikelgröße (LKS): | 440 nm |
| Viskosität (Viskosimeter, 23°C): | 84 mPas |
| pH (23°C): | 6,91 |

### Beispiel 6: Polyurethan-Dispersion 6

1072 g PolyTHF^{®} 2000, 407,6 g PolyTHF^{®} 1000, 827 g Desmophen^{®} C2200 und 48,1 g Polyether LB 25 wurden in einer Standard-Rührapparatur auf 70°C aufgeheizt. Anschließend wurde bei 70°C innerhalb von 5 min ein Gemisch aus 257,4 g Hexamethylendiisocyanat und 340 g Isophorondiisocyanat zugegeben und bei 120°C gerührt, bis der theoretische NCO-Wert erreicht oder leicht unterschritten war. Das fertige Prepolymer wurde mit 4820 g Aceton gelöst und dabei auf 50°C abgekühlt und anschließend wurde eine Lösung aus 27,3 g Ethylendiamin, 126,5 g Isophorondiamin, 67,0 g Diaminosulfonat und 1090 g Wasser innerhalb von 10 min zudosiert. Die Nachrührzeit betrug 10 min. Dann wurde durch Zugabe von 1180 g Wasser dispergiert. Es folgte die Entfernung des Lösemittels durch Destillation im Vakuum.

Die erhaltene weiße Dispersion hatte nachfolgenden Eigenschaften

| | |
|---|---|
| Feststoffgehalt: | 60% |
| Partikelgröße (LKS): | 312 nm |
| Viskosität (Viskosimeter, 23°C): | 286 mPas |
| pH (23°C): | 7,15 |

### Beispiele 7-12: Schäume, hergestellt aus den Polyurethan-Dispersionen der Beispiele 1-6

Die in der Tabelle 1 angegebenen Mengen der Polyurethan-Dispersionen, hergestellt wie in den Beispielen 1-6 beschrieben, wurden mit den Schaumhilfsmitteln wie dort ebenso angegeben vermischt und unter Verwendung eines handelsüblichen Handrührgerätes (Rührer aus gebogenem Draht) auf 1 Liter Schaumvolumen aufgeschlagen. Unter weiterem Rühren wurden die erhaltenen Schäume durch Zugabe von Praestol^{®} 185 K schließlich koaguliert; durch die Koagulation blieb das Schaumvolumen unverändert (leichte Viskositätszunahme). Danach wurden die Schäume mittels eines Filmziehgerätes (Rakel), Spalthöhe in der Tabelle 1 angegeben, auf siliconbeschichtetes Papier aufgezogen. In der Tabelle 1 sind ebenso die Trocknungsbedingungen der wie angegeben hergestellten Schäume aufgeführt. Es wurden durchweg reinweiße Schäume mit guten mechanischen Eigenschaften und einer feinen Porenstruktur erhalten.

**Tabelle 1**

| | Menge [g] | | | | | |
|---|---|---|---|---|---|---|
| Schaum Nr. | Polyurethan-Dispersion (Beispiel) | Stokal^{®} STA | Stokal^{®} SR | Praestol ^{®}185 k | SH¹⁾ [mm] | Aushärtung |
| 1a | 235,0 (1) | 4,2 | 5,6 | 5,0 | 2 | 2h/37°C |
| 1b | 235,0(1) | 4,2 | 5,6 | 5,0 | 4 | 18h/37°C |
| 1c | 235,0 (2) | 4,2 | 5,6 | 5,0 | 6 | 18h/37°C |
| 1d | 235,0 (2) | 4,2 | 5,6 | 5,0 | 4 | 18h/37°C,30min/120°C |
| 1e | 235,0 (2) | 4,2 | 5,6 | 5,0 | 6 | 18h/37°C,30min/120°C |
| 2 | 235,0 (2) | 4,2 | 5,6 | 5,0 | 4 | 2 h /37°C, 30 min /120°C |
| 3 | 235,0 (3) | 4,2 | 5,6 | 5,0 | 4 | 18h/37°C |
| 4 | 235,0(4) | 4,2 | 5,6 | 5,0 | 4 | 2h/37°C,30min/120°C |
| 5 | 235,0 (5) | 4,2 | 5,6 | 5,0 | 4 | 2 h / 37°C, 30 min /120°C |
| 6 | 235,0 (6) | 4,2 | 5,6 | 5,0 | 4 | 2 h / 37°C, 30 min /120°C |

| | | | | | | |
|---|---|---|---|---|---|---|
| ¹⁾ Spalthöhe Rakel | | | | | | |

Wie der Tabelle 2 zu entnehmen ist, zeigten allen Schäume eine sehr schnelle Aufnahme von Wasser, eine hohe Absorption von physiologischer Salzlösung ("Saugleistung bei freier Quellmöglichkeit"), eine sehr gute Wasserdampfdurchlässigkeit ("MVTR") und darüber hinaus eine gute mechanische Festigkeit, insbesondere auch nach Feuchtelagerung.

**Tabelle 2**

| Schaum Nr. | Aufnahmegeschwindigkeit¹⁾ [s] | Freie Saugleistung²) [g/100 cm²] | MVTR³⁾ [g/m²*24h] |
|---|---|---|---|
| 1a | nicht bestimmt | 13,4 | 6500 |
| 1b | nicht bestimmt | 23,6 | 6300 |
| 1c | nicht bestimmt | 33,0 | 5100 |
| 1d | 9 | 20,1 | 4400 |
| 1e | 9 | 29,6 | 4200 |
| 2 | 7 | 21,4 | 4100 |
| 3 | 7 | 23,4 | 3700 |
| 4 | 18 | 20,2 | 4100 |
| 5 | 11 | 25,8 | 4300 |
| 6 | 17 | 22,1 | 4400 |

| | | | |
|---|---|---|---|
| 1) Zeit bis zum vollständigen Eindringen eines Tropfens destilliertes Wasser in den Schaum (Prüfung der zum Papier gerichteten Seite); ²⁾ Absorption physiologischer Salzlösung bestimmt nach DIN EN 13726-I, Teil 3.2 (5 anstelle von 9 Prüfmustern); ³⁾ "moisture vapour transition rate" (Wasserdampfdurchlässigkeit) bestimmt nach DIN EN 13726-2, Teil 3.2 | | | |

### Beispiel 13:

54 g einer nach Beispiel 2 hergestellten Polyurethan-Dispersion wurden mit 1,37 g Simulsol^{®} SL 26 vermischt. Diese Mischung wurde in eine Kammer einer geeigneten 2-Komponenten-Aerosol-Dose gegeben; die andere Kammer wurde mit 1,69 g Praestol^{®} 185 K gefüllt. Die Komponenten wurden schließlich mit 6 g einer Treibmittelmischung aus i-Butan/Propan/n-Butan versetzt. Nach dem Sprühen (ca. 1 cm Nassfilmdicke) und Trocknen bei Umgebungsbedingungen wurde ein reinweißer, feinzelliger Schaum erhalten.

## Patentansprüche

1. Biomedizinischer Schaumartikel für den Wundbereich, erhältlich durch das Aufsprühen einer Zusammensetzung umfassend mindestens eine ionische Polymerdispersion oder - emulsion ,mindestens ein Koagulans sowie einem Schaumhilfsmittel direkt auf die Haut, insbesondere auf eine Wunde.

2. Biomedizinischer Schaumartikel gemäß Anspruch 1, **dadurch gekennzeichnet, dass** die ionische Polymerdispersion oder -emulsion ausgewählt ist aus ionischen Kautschuk-Latex-Dispersionen, ionischen Polyurethan-Dispersionen, Dispersionen ionischer (Meth)acrylat-Copolymere sowie Dispersionen natürlich vorkommender ionischer Biopolymere auf Kohlenhydrat-Basis wie Cellulosederivate, z. B. Celluloseacetatphthalat (CAP), Celluloseacetatsuccinat (CAS), Celluloseacetattrimelitat (CAT), Hydroxypropylmethylcellulosephthalat (HPMCP), Carboxymethylcellulose (CMC), Chitosan, auch Chitin, Hyaluronan, Dextrin, Cellulose oder Stärke sowie weiterer natürlicher Biopolymere wie z.B. Lignin oder Casein.

3. Biomedizinischer Schaumartikel gemäß Anspruch 1 oder 2, **dadurch gekennzeichnet, dass** die ionische Polymerdispersion oder -emulsion ausgewählt ist aus wässrigen Polyurethan -Dispersionen, aliphatischen Polyurethan-Dispersionen und Polyurethan-Hybrid-Emulsionen.

4. Biomedizinischer Schaumartikel gemäß einem der Ansprüche 1 bis 3, **dadurch gekennzeichnet, dass** die ionische Polymerdispersion oder -emulsion eine wässrige, anionische, hydrophile Polyurethan -Dispersion ist.

5. Biomedizinischer Schaumartikel gemäß einem der Ansprüche 1 bis 3, **dadurch gekennzeichnet, dass** die ionische Polymerdispersion oder -emulsion eine wässrige, anionische, hydrophile Polyurethan -Dispersion ist, die **dadurch** erhältlich ist, indem
A) isocyanatfunktionelle Prepolymere aus
A1) organischen Polyisocyanaten
A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
A3) gegebenenfalls hydroxyfunktionellen Verbindungen mit Molekulargewichten von 62 bis 399 g/mol und
A4) gegebenenfalls isocyanatreaktiven, anionischen oder potentiell anionischen und/oder gegebenenfalls nichtionischen Hydrophilierungsmitteln, hergestellt werden,
B) deren freie NCO-Gruppen dann ganz oder teilweise
B1) gegebenenfalls mit aminofunktionellen Verbindungen mit Molekulargewichten von 32 bis 400 g/mol und
B2) mit isocyanatreaktiven, bevorzugt aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

6. Biomedizinischer Schaumartikel gemäß Anspruch 5, **dadurch gekennzeichnet, dass** bei der Herstellung der wässrigen, anionisch hydrophilierten Polyurethan-Dispersionen (I) in A1) 1,6-Hexamethylendiisocyanat, Isophorondiisocyanat, die isomeren Bis-(4,4'-isocyanatocyclohexyl)methane, sowie deren Mischungen eingesetzt werden und in A2) eine Mischung aus Polycarbonatpolyolen und Polytetramethylenglykolpolyolen eingesetzt wird, wobei der Anteil der Summe der Polycarbonat- und Polytetramethylenglykolpolyetherpolyole an der Komponente A2) mindestens 70 Gew.-% beträgt.

7. Biomedizinischer Schaumartikel gemäß Anspruch 5 oder 6, **dadurch gekennzeichnet, dass** das kationische Koagulans (II) ein Copolymer des Acrylamids ist, welches Struktureinheiten der allgemeinen Formel (1) und (2) aufweist 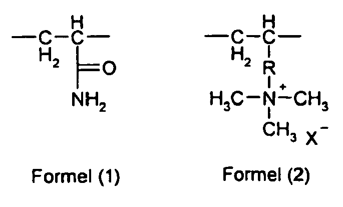 wobei
R C=O, -COO(CH₂)₂- oder -COO(CH₂)₃- und
X⁻ ein Halogenidion ist.

8. Biomedizinischer Schaumartikel gemäß einem der Ansprüche 1 bis 7, **dadurch gekennzeichnet, dass** die ionische Polymerdispersion oder -emulsion zusätzlich mindestens einen Wirkstoff ausgewählt aus der Gruppe der Antiseptika, Wachstumsfaktoren, Proteaseinhibitoren und nichtsteroidale Antiphlogistika/Opiate enthält.

## Claims

1. Biomedical foam article for the wound sector, obtainable by spraying a composition comprising at least one ionic polymeric dispersion or emulsion, at least one coagulant and also a foam auxiliary directly onto the skin, in particular onto a wound.

2. Biomedical foam article according to Claim 1, **characterized in that** the ionic polymeric dispersion or emulsion is selected from ionic rubber latex dispersions, ionic polyurethane dispersions, dispersions of ionic (meth)acrylate copolymers and dispersions of naturally occurring ionic biopolymers based on carbohydrate such as cellulose derivatives, for example cellulose acetate phthalate (CAP), cellulose acetate succinate (CAS), cellulose acetate trimelitate (CAT), hydroxypropylmethylcellulose phthalate (HPMCP), carboxymethylcellulose (CMC), chitosan, as well as chitin, hyaluronan, dextrin, cellulose or starch and also further natural biopolymers such as, for example, lignin or casein.

3. Biomedical foam article according to Claim 1 or 2, **characterized in that** the ionic polymeric dispersion or emulsion is selected from aqueous polyurethane dispersions, aliphatic polyurethane dispersions and polyurethane hybrid emulsions.

4. Biomedical foam article according to any one of Claims 1 to 3, **characterized in that** the ionic polymeric dispersion or emulsion is an aqueous anionic hydrophilic polyurethane dispersion.

5. Biomedical foam article according to any one of Claims 1 to 3, **characterized in that** the ionic polymeric dispersion or emulsion is an aqueous anionic hydrophilic polyurethane dispersion obtainable by
A) isocyanate-functional prepolymers being prepared from
A1) organic polyisocyanates
A2) polymeric polyols having number average molecular weights in the range from 400 to 8000 g/mol, preferably in the range from 400 to 6000 g/mol and more preferably in the range from 600 to 3000 g/mol and OH functionalities in the range from 1.5 to 6, preferably in the range from 1.8 to 3 and more preferably in the range from 1.9 to 2.1, and
A3) optionally hydroxyl-functional compounds having molecular weights in the range from 62 to 399 g/mol, and
A4) optionally isocyanate-reactive, anionic or potentially anionic and/or optionally nonionic hydrophilicizing agents,
and
B) their free NCO groups then being wholly or partly reacted
B1) optionally with amino-functional compounds having molecular weights in the range from 32 to 400 g/mol, and
B2) with isocyanate-reactive, preferably amino-functional, anionic or potentially anionic hydrophilicizing agents
by chain extension, and the prepolymers being dispersed in water before, during or after step B), any potentially ionic groups present being converted into the ionic form by partial or complete reaction with a neutralizing agent.

6. Biomedical foam article according to Claim 5, **characterized in that** the aqueous, anionically hydrophilicized polyurethane dispersions (I) are prepared using in A1) 1,6-hexamethylene diisocyanate, isophorone diisocyanate, the isomeric bis(4,4'-isocyanatocyclohexyl)methanes, and also mixtures thereof, and in A2) a mixture of polycarbonate polyols and polytetramethylene glycol polyols, the proportion of component A2) which is contributed by the sum total of the polycarbonate and polytetramethylene glycol polyether polyols being at least 70% by weight.

7. Biomedical foam article according to Claim 5 or 6, **characterized in that** the cationic coagulant (II) is an acrylamide copolymer comprising structural units of the general formula (1) and (2) 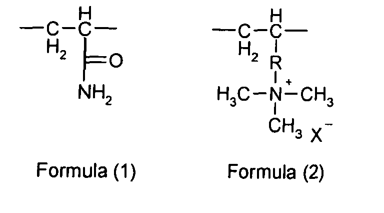 where
R is C=O, -COO(CH₂)₂- or -COO(CH₂)₃-, and
X- is a halide ion.

8. Biomedical foam article according to any one of Claims 1 to 7, **characterized in that** the ionic polymeric dispersion or emulsion additionally contains at least one active component selected from the group consisting of antiseptics, growth factors, protease inhibitors and non-steroidal anti-inflammatories/opiates.

## Revendications

1. Article moussant biomédical destiné à la zone blessée, réalisable par pulvérisation d'une composition comprenant au moins une dispersion ou une émulsion ionique de polymère, au moins un coagulant ainsi qu'un agent moussant directement sur la peau, en particulier sur une plaie.

2. Article moussant biomédical selon la revendication 1, **caractérisé en ce que** la dispersion ou l'émulsion ionique de polymère est sélectionnée parmi des dispersions ioniques de latex de caoutchouc, des dispersions ioniques de polyuréthane, des dispersions de copolymères ioniques de (méth)acrylate ainsi que des dispersions de biopolymères ioniques existant naturellement à base d'hydrates de carbone comme des dérivés de la cellulose, par exemple le phtalate d'acétate de cellulose (PAC), le succinate d'acétate de cellulose (SAC), le trimélitate d'acétate de cellulose (TAC), le phtalate d'hydroxypropylméthylcellulose (PHPMC), la carboxyméthylcellulose (CMC), le chitosane, aussi la chitine, le hyaluronane, la dextrine, la cellulose ou l'amidon ainsi que d'autres biopolymères naturels comme par exemple la lignine ou la caséine.

3. Article moussant biomédical selon la revendication 1 ou 2, **caractérisé en ce que** la dispersion ou l'émulsion ionique de polymère est sélectionnée parmi des dispersions aqueuses de polyuréthane, des dispersions de polyuréthane aliphatiques et des émulsions hybrides de polyuréthane.

4. Article moussant biomédical selon l'une quelconque des revendications 1 à 3, **caractérisé en ce que** la dispersion ou l'émulsion ionique de polymère est une dispersion aqueuse, anionique, hydrophile de polyuréthane.

5. Article moussant biomédical selon l'une quelconque des revendications 1 à 3, **caractérisé en ce que** la dispersion ou l'émulsion ionique de polymère est une dispersion aqueuse, anionique, hydrophile de polyuréthane, qui peut être obtenue **en ce que**
A) on fabrique des prépolymères à fonctions isocyanates à partir de
A1) polyisocyanates organiques
A2) polyols polymères avec des poids moléculaires moyens de 400 à 8000 g/mole, de préférence de 400 à 6000 g/mole, et de préférence encore de 600 à 3000 g/mole, et des fonctionnalités OH de 1,5 à 6, de préférence de 1,8 à 3, de préférence encore de 1,9 à 2,1, et
A3) éventuellement de composés à fonctions hydroxy avec des poids moléculaires de 62 à 399 g/mole, et
A4) éventuellement des agents d'hydrophilisation à réaction isocyanate, anioniques ou potentiellement anioniques et/ou éventuellement non ioniques,
B) dont les groupes NCO libres sont alors convertis totalement ou partiellement
B1) éventuellement avec des composés à fonctions aminées avec des poids moléculaires de 32 à 400 g/mole, et
B2) avec des agents d'hydrophilisation à réaction isocyanate, de préférence à fonctions aminées, anioniques ou potentiellement anioniques avec prolongation de chaînes, et les prépolymères sont dispersés dans l'eau avant, pendant ou après l'étape B), dans lequel des groupes potentiellement ioniques éventuellement présents sont convertis dans la forme ionique par conversion partielle ou totale avec un agent de neutralisation.

6. Article moussant biomédical selon la revendication 5, **caractérisé en ce que**, lors de la fabrication des dispersions aqueuses, anioniques hydrophiles (I) de polyuréthane, on utilise dans A1) le diisocyanete de 1,6-hexaméthylène, le diisocyanate d'isophorone, les isomères bis-(4,4'-isocyanatocyclohexyl)méthane, ainsi que leurs mélanges, et on utilise dans A2) un mélange de polyols de polycarbonates et de polyols de polytétraméthylèneglycol, dans lequel la part de la somme des polyols de polycarbonates et de polytétraméthylèneglygol dans le composant A2) vaut au moins 70% en poids.

7. Article moussant biomédical selon la revendication 5 ou 6, **caractérisé en ce que** le coagulant cationique (II) est un copolymère de l'acrylamide, qui présente des unités structurelles des formules générales (1) et (2) 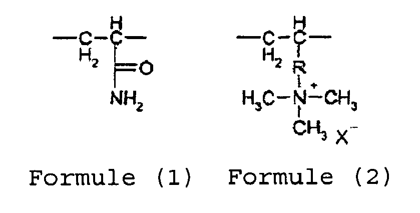 dans lesquelles
R est C=O, -COO(CH₂)₂- ou -COO(CH₂)₃- et
X- est un ion halogénure.

8. Article moussant biomédical selon l'une quelconque des revendications 1 à 7, **caractérisé en ce que** la dispersion ou l'émulsion ionique de polymère contient en plus au moins une substance sélectionnée dans le groupe des antiseptiques, des facteurs de croissance, des inhibiteurs de protéase et des antiphlogistiques/opiacés non stéroïdaux.
